(19) Europäisches Patentamt
European Patent Office
Office européen des brevets

(11) Veröffentlichungsnummer: **0 039 809**
**A2**

(12) # EUROPÄISCHE PATENTANMELDUNG

(21) Anmeldenummer: 81103116.0

(22) Anmeldetag: 25.04.81

(51) Int. Cl.³: C 07 D 277/82
A 61 K 31/425

(30) Priorität: 10.05.80 DE 3017976

(43) Veröffentlichungstag der Anmeldung:
18.11.81 Patentblatt 81/46

(84) Benannte Vertragsstaaten:
BE CH DE FR GB IT LI LU NL SE

(71) Anmelder: Dr. Karl Thomae GmbH
Postfach 720
D-7950 Biberach (Riss)(DE)

(72) Erfinder: Eberlein, Wolfgang, Dr.Dipl.-Chem.
Obere Au 6
D-7950 Biberach 1(DE)

(72) Erfinder: Trummlitz, Günter, Dr.Dipl.-Chem.
Buchenweg 27
D-7951 Warthausen 1(DE)

(72) Erfinder: Engel, Wolfhard, Dr.Dipl.-Chem.
Mozartstrasse 13
D-7950 Biberach 1(DE)

(72) Erfinder: Schmidt, Günther, Dr.Dipl.-Chem.
Johann-Sebastian-Bach-Strasse 27
D-7950 Biberach 1(DE)

(72) Erfinder: Engelhardt, Günther, Dr. Prof.
Unterer Bühl 18
D-7950 Biberach 1(DE)

(72) Erfinder: Zimmermann, Rainer, Dr.Dipl.-Biochem.
Laurenbühlstrasse 17
D-7951 Mittelbiberach(DE)

(54) **Substituierte 2-Amino-5-halogen-benzothiazole, Verfahren zu ihrer Herstellung und diese Verbindungen enthaltende Arzneimittel.**

(57) Beschrieben wird als neue Verbindung das 2-Amino-5-brom-6-methoxy-benzothiazol, ein Verfahren zu seiner Herstellung, des weiteren Arzneimittel, die diese Substanz oder aber auch die an sich bekannten Substanzen 2-Amino-5-chlor-4-methoxy-benzothiazol oder 2-Amino-5-chlor- 6-methoxy-benzothiazol enthalten. Die Wirksubstanzen zeigen eine sehr gute analgetische und antipyretische Wirkung.

EP 0 039 809 A2

COMPLETE DOCUMENT

Croydon Printing Company Ltd

Dr. Karl Thomae GmbH
Case 5/792
Dr. Bu./pf./st

Substituierte 2-Amino-5-halogen-benzothiazole, Verfahren zu ihrer Herstellung und diese Verbindungen enthaltende Arzneimittel

Die Erfindung betrifft

a) als neue Substanz
2-Amino-5-brom-6-methoxy-benzothiazol der Formel

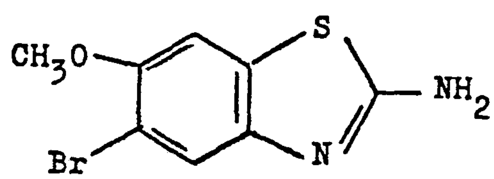

,(I)

des weiteren

b) Arzneimittel,
welche diese Substanz enthalten und

c) Arzneimittel,
welche die an sich bekannten Verbindungen 2-Amino-5-chlor-4-methoxy-benzothiazol und 2-Amino-5-chlor-6-methoxy-benzothiazol enthalten,
ferner Verfahren zur Herstellung dieser Verbindungen.

Es wurde überraschenderweise gefunden, daß die vorstehend genannten Verbindungen eine sehr gute analgetische und antiinflammatorische Wirkung bei guter Verträglichkeit zeigen.

Das 2-Amino-5-chlor-4-methoxy-benzothiazol ist als Zwischenprodukt in der DE-OS 2 656 468 genannt worden, allerdings sind dort keine Angaben über eine mögliche physiologische Wirkung dieser Verbindung enthalten. Dasselbe gilt für das 2-Amino-5-chlor-6-methoxy-benzothiazol, welches in Helv. Chem. Acta 63, 682 (1980) beschrieben ist.

Die obengenannten Verbindungen lassen sich wie folgt herstellen:

a) durch Umsetzung von substituierten Anilinen der allgemeinen Formel

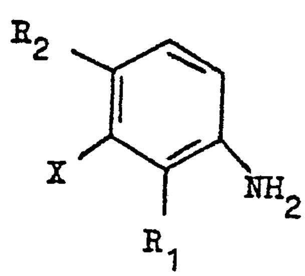

,(II)

in der entweder $R_1$ die Methoxygruppe und $R_2$ ein Wasserstoffatom oder $R_1$ ein Wasserstoffatom und $R_2$ die Methoxygruppe und X ein Chlor- oder Bromatom darstellt, mit Dirhodan und anschließende Cyclisierung der dabei entstehenden Thiocyanverbindungen der allgemeinen Formel

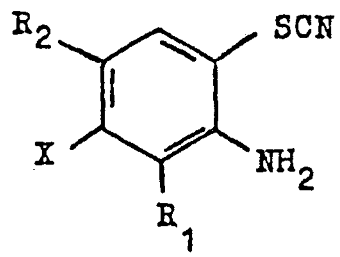

,(IIa)

zu dem entsprechend substituierten 2-Amino-5-halogen-benzothiazol. Die Thiocyanverbindungen der allgemeinen Formel IIa brauchen nicht vor der weiteren Cyclisierung isoliert zu werden.

Die Umsetzung von Verbindungen der allgemeinen Formel II mit Dirhodan erfolgt im allgemeinen in organischen Lösungsmitteln. Es werden dabei polare Lösungsmittel wie Eisessig, Ethanol, Methanol, bevorzugt. Das Dirhodan wird vorteilhafterweise in situ aus entsprechenden Salzen des Rhodanwasserstoffs, beispielsweise aus Natrium-, Kalium- oder Ammoniumrhodanid, durch Zugabe einer Lösung von Chlor oder Brom in demselben Lösungsmittel erzeugt. Die Reaktion erfolgt bei Temperaturen zwischen 10 und 25°C;

Die Cyclisierung einer Verbindung der Formel IIa erfolgt entweder spontan oder durch Zusatz einer Säure, beispielsweise durch Zugabe einer ethanolischen Chlorwasserstoff-Lösung, bei einer Temperatur von 20 bis 50°C, vorzugsweise von 25 bis 40°C.

b) Durch Halogenierung eines 2-Amino-benzothiazols der allgemeinen Formel

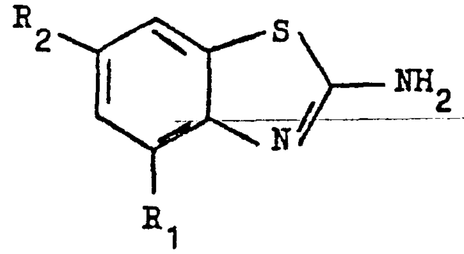

,(III)

in der $R_1$ die Methoxygruppe und $R_2$ ein Wasserstoffatom oder $R_2$ die Methoxygruppe und $R_1$ ein Wasserstoffatom darstellen.

Die Halogenierung erfolgt in einem inerten organischen Lösungsmittel, vorzugsweise in Eisessig, indem das Halogen nach und nach eingeleitet wird. Die Bromierung erfolgt vorzugsweise durch Zutropfen einer bromhaltigen Lösung desselben Lösungsmittels. Die Halogenierung läßt sich bei Temperaturen zwischen 0 und 60°C, vorzugsweise aber bei Zimmertemperatur durchführen;

c) durch dehydrierende Cyclisierung eines Thioharnstoffs der allgemeinen Formel,

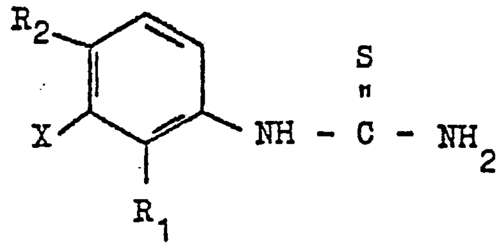

,(IV)

in der $R_1$, $R_2$ und X wie oben in Formel II definiert sind, mit Brom.
Die Umsetzung wird in einem organischen Lösungsmittel durchgeführt, beispielsweise in Eisessig, Ethanol, Methanol oder Chloroform. Die Umsetzung erfolgt bei Temperaturen zwischen 20°C und dem Siedepunkt des Reaktionsgemisches. Es empfiehlt sich, die Eintragung des Broms bei Temperaturen zwischen 20 und 40°C durchzuführen und das Reaktionsgemisch zur Vervollständigung der Reaktion schließlich bis zu dessen Siedepunkt zu erhitzen. Überschüssiges Brom wird nach der Reaktion, z.B. mit Hilfe von Natriumhydrogensulfit, entfernt.

Die Ausgangsverbindungen der allgemeinen Formel II sind literaturbekannt, dasselbe gilt für die Verbindungen der allgemeinen Formel III.

Die Ausgangsverbindungen der allgemeinen Formel IV erhält man beispielsweise aus einen Benzoylthioharnstoff der allgemeinen Formel

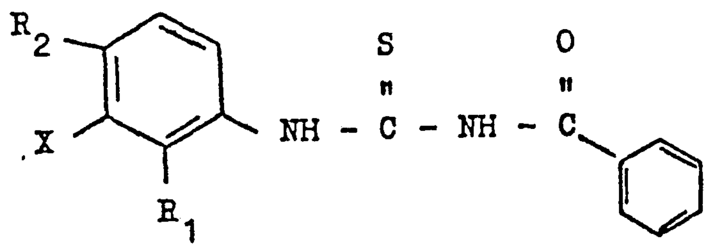

,(V)

durch Erhitzen einer z.B. alkoholischen Lösung einer Verbindung der allgemeinen Formel V in Gegenwart eines Alkalialkoholats, z.B. Natriummethylat. Die Benzoylthioharnstoffe der Formel V sind leicht zugänglich aus dem entsprechend substituierten Anilin, indem dieses mit einer Lösung von Ammoniumrhodanid und Benzoylchlorid in einem geeigneten Lösungsmittel, z.B. in Aceton, durch Erhitzen auf die Rückflußtemperatur des Lösungsmittels umgesetzt wird.

Die Verbindungen 2-Amino-5-chlor-6-methoxy-benzothiazol, 2-Amino-5-chlor-4-methoxy-benzothiazol und 2-Amino-5-brom-6-methoxy-benzothiazol zeigen eine sehr gute analgetische und antipyretische Wirkung und eignen sich deshalb sehr gut zur Herstellung entsprechender Arzneimittel. Ein weiterer Gegenstand dieser Erfindung bezieht sich auf die Bereitstellung von Arzneimitteln mit schmerzmindernder Wirkung.

Es wurde zum Beispiel das
2-Amino-5-chlor-6-methoxy-benzothiazol

auf seine analgetische Wirkung gegenüber dem Entzündungsschmerz der Ratte, auf seine analgetische Wirkung gegen den durch Wärme ausgelösten Schmerz der Maus, auf seine die Körpertemperatur der Ratte senkende Wirkung sowie auf die akute Toxizität an der Maus untersucht.

<u>Methodik:</u>

1) <u>Wirkung gegen den Entzündungsschmerz der Ratte</u>

Die Versuchsanordnung entsprach der von RANDALL-SELITTO (Arch. int. Pharmacodyn. <u>111</u>, 409 (1957) angegebenen.

Männliche Chbb:THOM-Ratten mit einem Gewicht zwischen 100 und 130 g erhielten eine subplantare Injektion von 0,1 ml einer 5%igen Suspension von Hefezellen in 5,55 %iger Glukoselösung in eine Hinterpfote. 1 1/2 bzw. 2 1/4 Stunden nach der Injektion des Phlogisticums erhielten die Tiere verschiedene Dosen der Prüfsubstanz als Verreibung in 1 %iger Methylzellulose (1 ml/100 g Tier) per Schlundsonde beigebracht. Kontrolltiere erhielten entsprechende Volumina des Vehikels. 90 bzw. 45 min nach Applikation der Prüfsubstanz (d.h. 3 Stunden nach der subplantaren Injektion der Hefesuspension) wurde bei den Ratten die Schmerzschwelle in g Auflagedruck/Pfote bestimmt.
Aus der nach den verschiedenen Dosen der Prüfsubstanz gemessenen Schmerzschwelle wurde nach linearer Regressionsanalyse nach LINDER (Statistische Methoden, 4. Aufl. pp. 148-162, Birkhäuser, Basel 1964) eine $ED_{50}$ mit den Vertrauensgrenzen nach FIELLER (Quart. J. Pharm. Pharmacol. 17, 117 (1944) als jene Dosis ermittelt, die eine Anhebung der Schmerzschwelle um 50 % gegenüber der der Kontrollen bewirkte.

2) Wirkung gegen den Wärmeschmerz der Maus

Die Prüfung erfolgte mit Hilfe einer Modifikation der Versuchsanordnung nach CHEN u. BECKMAN (Science 113, 631 (1951) an männlichen Chbb:NMRI(SPF)-Mäusen mit einem mittleren Gewicht von 20 g. Die "heiße Platte" bestand aus Aluminium und hatte eine Oberflächentemperatur von 52°C.

Die Prüfsubstanz wurde als Verreibung in 1 %iger Methylzellulose (0,1 ml/10 g Maus) per Schlundsonde beigebracht. Vor der Behandlung mit Prüfsubstanz wurden die Tiere im Abstand von 30 min. zweimal auf die heiße Platte gesetzt. Es wurde ihre individuelle Reaktionszeit ermittelt. Nach Gabe der Prüfsubstanz wurden in Abständen von 30 min. die Reaktionszeit erneut gemessen.

Aus der mit den verschiedenen Dosen der Prüfsubstanz erzielten gemittelten maximalen Verlängerung der Reaktionszeit wurde nach linearer Regressionsanalyse nach LINDER (s.o.) eine $ED_{100}$ mit den Vertrauensgrenzen nach FIELLER (s.o.) als die Dosis berechnet, die zu einer 100 %igen Verlängerung der Reaktionszeit führte.

3. Wirkung auf die Körpertemperatur der Ratte

Die Prüfung der temperatursenkenden Wirkung erfolgte durch die Kontrolle des Verlaufes der rektal gemessenen Körpertemperatur von Chbb:THOM-Ratten mit einem Gewicht zwischen 125-150 g über im Rektum verweilende Thermoelemente. Die Prüfsubstanz wurde als Verreibung in 1 %iger Methylzellulose (1,0 ml/100 g Tier) per Schlundsonde beigebracht. Aus den nach Gabe der verschiedenen Dosen der Prüfsubstanz gewonnenen Werten für die mittlere maximale Temperatursenkung wurde nach linearer Regressionsanalyse nach LINDER (s.o.) eine $ED_{-1,5^{\circ}C}$ als jene Dosis berechnet, die eine Senkung der Körpertemperatur um 1,5°C bewirkte.

4. Akute Toxizität an der Maus

Die Bestimmung der akuten Toxizität erfolgte an Chbb:NMRI (SPF)-Mäusen beider Geschlechter mit einem mittleren Gewicht von 20 g. Die Prüfsubstanz wurde als Verreibung in 1 %iger Methylzellulose (0,5 ml/10 g Tier) per Schlundsonde verabfolgt. Die Berechnung der $LD_{50}$ erfolgte (soweit möglich) nach LITCHFIELD u. WILCOXON (J. Pharmacol. exp. Therap. 96, 99 (1949)) aus dem Prozentsatz der Tiere, die nach den verschiedenen Dosen innerhalb von 14 Tagen verstarben.

Ergebnisse:

Die bei der pharmakologischen Prüfung erhobenen Befunde sind in der nachfolgenden Tabelle zusammengestellt. Die geprüfte Verbindung zeichnet sich bei einer sehr geringen Toxizität durch gute analgetische und antipyretische Wirkungsqualitäten aus.

Pharmakologische Wirksamkeit nach oraler Gabe

| Test | ED bzw. $LD_{50}$ mg/kg | Vertrauensgrenzen bei 95 % Wahrscheinlichkeit |
|---|---|---|
| Analgesie Ratte nach RANDALL-SELITTO | | |
| 45 min nach Appl. [$ED_{50}$] | 79 | 74 - 84 |
| 90 min nach Appl. [$ED_{50}$] | 80 | 76 - 84 |
| Analgesie Maus "hot plate" [$ED_{100}$] | 144 | 53 - 202 |
| Körpertemperatursenkg. Ratte [$ED_{-1,5^\circ C}$] | 101 | 78 - 146 |
| Akute Toxizität Maus [$LD_{50}$] | 3050 | 2180 - 4270 |

Die nachfolgenden Beispiele sollen die Erfindung näher erläutern:

Beispiel 1

2-Amino-5-chlor-6-methoxy-benzothiazol

80 g (0,5 Mol) 3-Chlor-4-methoxy-anilin werden in 500 ml Eisessig gelöst und bei Raumtemperatur mit einer Lösung von 76 g (1 Mol) Ammoniumrhodanid in 250 ml Eisessig versetzt. Zu dieser Lösung tropft man bei 12 - 17°C innerhalb von einer Stunde die Lösung von 160 g (1 Mol) Brom in 125 ml Eisessig, rührt eine Stunde in der Kälte nach und saugt den erhaltenen Niederschlag ab. Das Rohprodukt wird mit wenig Eisessig gewaschen, in 2 Liter Wasser aufgeschlämmt und die erhaltene Suspension bei 30°C durch Zugabe von verdünnter Natronlauge alkalisch gestellt. Man saugt erneut ab, wäscht das Festprodukt mit Wasser und absolutem Alkohol und trocknet im Vakuum. Auf diese Weise erhält man 100 g (93 % der Theorie) 2-Amino-5-chlor-6-methoxy-benzothiazol von Schmelzpunkt 240 - 241°C.

NMR-Spektrum (DMSO/$CD_3OD$): 4,05 ppm (3H), 7,6 ppm (1H), 7,7 ppm (1H)

Beispiel 2

2-Amino-5-chlor-4-methoxy-benzothiazol

2.1 1-(3-Chlor-2-methoxy-phenyl)-3-benzoyl-thioharnstoff

14,0 g (0,1 Mol) Benzoylchlorid werden in 70 ml Aceton gelöst und bei 15 - 20°C mit einer Lösung von 7,6 g (0,1 Mol) Ammoniumrhodanid in 70 ml Aceton versetzt. Zu dieser Lösung tropft man innerhalb von 20 Minuten die

Lösung von 15,7 g (0,1 Mol) 2-Methoxy-3-chlor-anilin in 60 ml Aceton, erhitzt 4 Stunden unter Rückfluß, gießt den Ansatz auf Eis und erhält als Zwischenprodukt 28,2 g (88 % der Theorie) des gewünschten 3-Benzoyl-thioharnstoff der ohne weitere Reinigung nach Beispiel 2.2 weiter umgesetzt wird.

2.2 16.0 g (0,05 Mol) des nach Beispiel 2.1 hergestellten Thioharnstoffs werden in 400 ml Methanol suspendiert und mit 6 g Natriummethylat versetzt. Man rührt anschließend eine Stunde bei Raumtemperatur nach, versetzt mit 2n-Salzsäure bis zum pH-Wert 4, engt im Vakuum ein und verrei den öligen Rückstand mit Ligroin. Man saugt ab, wäscht das Festprodukt mit Wasser und trocknet im Vakuum bei 70°C Auf diese Weise erhält man 8,7 g (81 % der Theorie) 1-(3-Chlor-2-methoxy-phenyl)-thioharnstoff vom Schmelzpunkt 117 - 118°C.

2.3 <u>2-Amino-5-chlor-4-methoxy-benzothiazol</u>

7,6 g (0,035 Mol) des nach Beispiel 2.2 hergestellten Thioharnstoffs werden in 70 ml Chloroform gelöst und bei 20 - 30°C innerhalb von 5 Minuten mit einer Lösung von 5,7 g Brom in 10 ml Chloroform versetzt. Unter starker Bromwasserstoffentwicklung wird 45 Minuten zum Rückfluß erhitzt. Man läßt abkühlen, saugt den Niederschlag ab und rührt ihn zunächst mit Natriumhydrogensulfit Lösung und anschließend mit 2n-Natronlauge aus. Der erneut gesammelte Niederschlag wird mit Wasser gewaschen und im Vakuum getrocknet. Man erhält 4,6 g (61 % der Theorie) 2-Amino-5-chlor-4-methoxy-benzothiazol vom Schmelzpunkt 185 - 187°C.

## Beispiel 3

### 2-Amino-5-brom-6-methoxy-benzothiazol

18 g (0,1 Mol) 2-Amino-6-methoxy-benzothiazol werden in 200 ml Eisessig gelöst. Zu dieser Lösung tropft man innerhalb einer Stunde eine Lösung von 18 g (0,11 Mol) Brom in 100 ml Eisessig, rührt eine Stunde nach, saugt vom ausgefallenen Hydrobromid ab und wäscht mit Eisessig nach. Der Niederschlag wird in 2 ltr. Wasser suspendiert und erneut vom Unlöslichen abfiltriert. Das Filtrat wird durch Zugabe einer 30 %igen Natriumhydroxid-Lösung alkalisch gestellt. Das ausgefallene Festprodukt wird abgesaugt und im Vakuum getrocknet. Durch chromatographische Reinigung an Kieselgel (Chloroform/Essigester = 1:1) erhält man 13 g (50 % der Theorie) der gewünschten Verbindung, welche nach dem Umkristallisieren aus Essigester bei 226-227°C schmilzt.

Die Verbindungen 2-Amino-5-chlor-4-methoxybenzothiazol, 2-Amino-5-brom-6-methoxy-benzothiazol und 2-Amino-5-chlor-4-methoxy-benzothiazol lassen sich in die üblichen pharmazeutischen Zubereitungsformen, wie Tabletten, Dragées, Suppositorien, Kapseln oder Säfte einarbeiten. Die Einzeldosierung beträgt hierbei für Erwachsene 20 bis 600 mg, vorzugsweise 50 bis 300 mg, dies entspricht einer Tagesdosierung von 60 bis 1800 mg, vorzugsweise von 180 bis 900 mg.

Die nachfolgenden Beispiele sollen die Herstellung einiger pharmazeutischer Zubereitungsformen verdeutlichen.

Beispiel I

Tabletten mit 50 mg 2-Amino-5-chlor-6-methoxy-benzothiazol

Zusammensetzung:
1 Tablette enthält:

| | |
|---|---|
| Wirkstoff | 50,0 mg |
| Milchzucker | 128,0 mg |
| Kartoffelstärke | 40,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 50 mg 2-Amino-5-chlor-6-methoxy-benzothiazol

Die nach Beispiel I hergestellten Tabletten werden nach bekannten Verfahren mit einer Hülle überzogen, die im wesentlichen aus Zucker und Talkum besteht. Die fertigen Dragées

werden mit Hilfe von Bienenwachs poliert.

Dragéegewicht: 300 mg

Beispiel III

Kapseln mit 60 mg 2-Amino-5-chlor-6-methoxy-benzothiazol

Zusammensetzung:

1 Kapsel enthält:

| | |
|---|---|
| Wirkstoff | 60,0 mg |

Herstellungsverfahren:

Der Wirkstoff wird mikronisiert und in Kapseln abgefüllt, letztere werden anschließend verschlossen.

Beispiel IV

Suppositorien mit 60 mg 2-Amino-5-chlor-6-methoxy-benzothiazol

Zusammensetzung:

1 Zäpfchen enthält:

| | |
|---|---|
| Wirkstoff | 60,0 mg |
| Zäpfchenmasse (z.B. Witepsol W 45 R) | 1 640,0 mg |
| | 1 700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.

Zäpfchengewicht: 1,7 g

Beispiel V

Tabletten zu 200 mg 2-Amino-5-chlor-4-methoxy-benzothiazol

1 Tablette enthält:

| | |
|---|---|
| Wirksubstanz | 200,0 mg |
| Milchzucker | 120,0 mg |
| Maisstärke | 70,0 mg |
| Polyvinylpyrrolidon | 8,0 mg |
| Magnesiumstearat | 2,0 mg |
| | 400,0 mg |

Herstellung:

Die Wirksubstanz, der Milchzucker und die Maisstärke werden mit einer wäßrigen Lösung von Polyvinylpyrrolidon gleichmäßig befeuchtet, durch ein Sieb mit 2 mm-Maschenweite gesiebt und im Umlufttrockenschrank bei 50°C getrocknet. Nach erneuter Siebung durch 1,5 mm-Maschenweite wird Magnesiumstearat zugemischt und die Mischung zu Tabletten verpreßt.

Tablettengewicht: 400 mg
Durchmesser: 11 mm, rund, biplan, beidseitige Facette und einseitige Teilkerbe.

Beispiel VI

Suppositorien zu 200 mg 2-Amino-5-chlor-4-methoxy-benzothiazol

1 Zäpfchen enthält:

| | |
|---|---|
| Wirksubstanz | 0,20 g |
| Hartfett (z.B. Witepsol H 19 oder Witepsol W 45) | 1,50 g |
| | 1,70 g |

Herstellung:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.
Zäpfchengewicht: 1,7 g.

Beispiel VII

Suspension mit 200 mg 2-Amino-5-chlor-6-methoxy-benzothiazol

100 ml Suspension enthalten:

| | | |
|---|---|---|
| Wirksubstanz | | 4,0 g |
| Carboxymethylcellulose | | 0,1 g |
| p-Hydroxybenzoesäuremethylester | | 0,05g |
| p-Hydroxybenzoesäurepropylester | | 0,01g |
| Rohrzucker | | 10,0 g |
| Glycerin | | 5,0 g |
| Sorbitlösung 70 % | | 20,0 g |
| Aroma | | 0,3 g |
| Wasser dest. | ad. | 100,0 ml |

Herstellungsverfahren:

Dest. Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren die Wirksubstanz zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 200 mg Wirksubstanz.

# P a t e n t a n s p r ü c h e

1. Als neue Verbindung 2-Amino-5-brom-6-methoxy-benzothiazol.

2. Arzneimittel, enthaltend 2-Amino-5-brom-6-methoxy-benzothiazol gemäß Anspruch 1, neben den üblichen Träger- und/oder Hilfsstoffen.

3. Arzneimittel, enthaltend das 2-Amino-5-chlor-6-methoxy-benzothiazol neben den üblichen Träger- und/oder Hilfsstoffen.

4. Arzneimittel, enthaltend das 2-Amino-5-chlor-4-methoxy-benzothiazol neben den üblichen Träger- und/oder Hilfsstoffen.

5. Verfahren zur Herstellung des neuen 2-Amino-5-brom-6-methoxy-benzothiazols der Formel

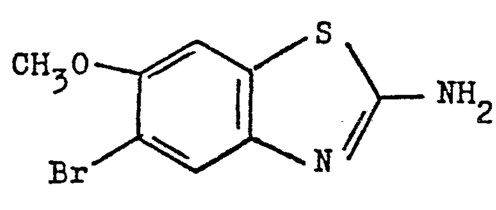
,(I)

dadurch gekennzeichnet, daß das 2-Amino-6-methoxy-benzothiazol in einem inerten organischen Lösungsmittel bei Temperaturen zwischen 0 und 60°C bromiert wird.